# EUROPEAN PATENT SPECIFICATION

(11) **EP 1 635 806 B1**
(45) Date of publication and mention of the grant of the patent: **28.05.2008**
(21) Application number: 04776447.7
(22) Date of filing: 10.06.2004
(51) Int. Cl.: A61K 31/16, A61K 31/22, A61K 31/7024, A61P 1/16

(54) **METHOD FOR TREATING ALCOHOLIC HEPATITIS**
VERFAHREN ZUR BEHANDLUNG VON ALKOHOLISCHER HEPATITIS
METHODE DE TRAITEMENT DE L'HEPATITE ALCOOLIQUE

(30) Priority: 13.06.2003 US 478637 P; 02.09.2003 US 499552 P
(43) Date of publication of application: 22.03.2006
(73) Proprietor: University of Pittsburgh of the Commonwealth System of Higher Education, Pittsburgh, PA 15260 (US)
(72) Inventor: FINK, Mitchell, P., Pittsburgh, PA 15238 (US); YANG, Runkuan, Pittsburgh, PA 15206 (US)
(74) Representative: Stevens, Ian Edward
(86) International application number: PCT/US2004/018490
(87) International publication number: WO 2005/002563

(56) References cited:
- WO-A-01/24793
- WO-A-02/074301
- WO-A-03/088955
- UESUGI TAKEHIKO ET AL: "Role of lipopolysaccharide-binding protein in early alcohol-induced liver injury in mice" JOURNAL OF IMMUNOLOGY, vol. 168, no. 6, 15 March 2002 (2002-03-15), pages 2963-2969, XP002309832 ISSN: 0022-1767
- NAVEAU SYLVIE ET AL: "Tumor necrosis factor soluble receptor p55 and lipid peroxidation in patients with acute alcoholic hepatitis" AMERICAN JOURNAL OF GASTROENTEROLOGY, vol. 96, no. 12, December 2001 (2001-12), pages 3361-3367, XP002309833 ISSN: 0002-9270
- NANJI AMIN A: "Role of Kupffer cells in alcoholic hepatitis" ALCOHOL, vol. 27, no. 1, May 2002 (2002-05), pages 13-15, XP002309834 ISSN: 0741-8329
- YANG R ET AL: "ETHYL PYRUVATE MODULATES INFLAMMATORY GENE EXPRESSION IN MICE SUBJECTED TO HEMORRHAGIC SHOCK" AMERICAN JOURNAL OF PHYSIOLOGY: GASTROINTESTINAL AND LIVER PHYSIOLOGY, AMERICAN PHYSIOLOGICAL SOCIETY, US, vol. 283, 2002, pages G212-G221, XP008019310 ISSN: 0193-1857
- ANTOSIEWICZ J ET AL: "Pyruvate protects cerulein induced acute pancreatitis" FREE RADICAL BIOLOGY AND MEDICINE, ELSEVIER SCIENCE, XX, vol. 29, no. Supplement 1, 2000, page S28, XP002262994 ISSN: 0891-5849
- YANG RUNKUAN ET AL: "Ethyl pyruvate ameliorates acute alcohol-induced liver injury and inflammation in mice." THE JOURNAL OF LABORATORY AND CLINICAL MEDICINE. NOV 2003, vol. 142, no. 5, November 2003 (2003-11), pages 322-331, XP009041310 ISSN: 0022-2143

### SEQUENCE LISTING

<110> Univeristy of Pittsburg of the Commonwealth System of Higher Education
   Fink, Mitchell P.
   Yang, Runkuan
<120> Method for Treating Alcoholic Hepatitis
<130> 3403.1002004
<150> 60/478,637
   <151> 2003-06-13
<150> 60/499,552
   <151> 2003-09-02
<160> 10
<170> FastSEQ for Windows Version 4.0
<210> 1
   <211> 22
   <212> DNA
   <213> Artificial Sequence
<220>
   <223> Primer for iNOS
<400> 1
   caccacaagg ccacatcgga tt 22
<210> 2
   <211> 22
   <212> DNA
   <213> Artificial Sequence
<220>
   <223> Primer for iNOS
<400> 2
   ttgttaccgt tgtagtccag cc 22
<210> 3
   <211> 25
   <212> DNA
   <213> Artificial.Sequence
<220>
   <223> Primer for TNF
<400> 3
   ggcaggtcta ctttggagtc attgc 25
<210> 4
   <211> 25
   <212> DNA
   <213> Artificial Sequence
<220>
   <223> Primer for TNF
<400> 4
   ggcttaagtg acctcggagc ttaca 25
<210> 5
   <211> 22
   <212> DNA
   <213> Artificial Sequence
<220>
   <223> Primer for IL-6
<400> 5
   ttccatccag ttgccttctt gg 22
<210> 6
   <211> 23
   <212> DNA
   <213> Artificial Sequence
<220>
   <223> Primer for IL-6
<400> 6
   gaccctttag cacctttact ctt 23
<210> 7
   <211> 24
   <212> DNA
   <213> Artificial Sequence
<220>
   <223> Primer for 18S ribosomal RNA
<400> 7
   cccggggagg tagtgacgaa aaat 24
<210> 8
   <211> 24
   <212> DNA
   <213> Artificial Sequence
<220>
   <223> Primer for 18S ribosomal RNA
<400> 8
   catcaaccta gaaccctcgc ccgc 24
<210> 9
   <211> 22
   <212> DNA
   <213> Artificial Sequence
<220>
   <223> Sense strand of doublestranded NF-KB oligonucleotide
<400> 9
   agttgagggg actttcccag gc 22
<210> 10
   <211> 22
   <212> DNA
   <213> Artificial Sequence
<220>
   <223> Sense strand of doublestranded NF-KB oligonucleotide
<400> 10
   gcctgggaaa gtcccctcaa ct 22

## Description

### BACKGROUND OF THE INVENTION

Alcoholic hepatitis is associated with considerable morbidity. The short-term mortality rate resulting from acute alcoholic hepatitis can be as high as 46% (Akriviadis et al., "Pentoxifylline improves short-term survival in severe acute alcoholic hepatitis: a double-blind placebo-controlled trial", Gastroenterology 119:1637-1648 (2000); and Akriviadis et al., "Failure of colchicines to improve short-term survival in patients with alcoholic hepatitis", Gastroenterology 99:811-8.18(1990)).

General measures for treatment of alcoholic hepatitis include abstinence from alcohol and supportive care such as nutritive support, relief of vitamin deficiencies and dietary adjustments if ascites or hepatic encephalopathy are present. While alcoholic hepatitis is reversible if the patient stops drinking, it usually takes several months to resolve. Thus, there is an urgent need for new methods of preventing and/or ameliorating the effects of alcoholic hepatitis.

WO 02/074301 describes a method of using pyrurate and/or its derivatives for the treatment of cytokine-mediated inflammatory conditions.

Uesugi et al (Journal of Immunology, vol. 168(6), 2002, pages 2963-2969) discloses the hypothesis that LPS-binding protein (LBP) may play an important role in early alcohol-induced liver injury by enhancing LPS-induced signal transduction.

Naveau et al (The American Journal of Gastroesterology, vol. 96(12), 2001, pages 3361 to 3367) describes that in patients with acute alcoholic hepatitis, TNF_{S}R_{P}55 probably mediates cytotoxicity of TNF-α, and that cytotoxic effect could be amplified by tGSH depletion in enhancing lipid peroxidation.

Nanji AA (Alcohol, Vol. 27, no. 1, 2002, pages 13 to 15) discloses that a combined role for tumor necrosis factor-alpha and cyclooxygenase-2 is important in the pathogenesis of alcoholic hepatitis.

Yang et al (Am J Physiol Gastrointe Liver Physiol, 283; G212-221, 2002) describes that ethyl pyrurate modulates inflammatory gene expression in mice subjected to hemorrhagic shock.

WO 01/24793 discloses a pyrurate ester composition and method of use for resuscitation after events of ischemia and reperfusion.

Antosiewicz et al (Free Radical Biology and Medicine, Vol. 29, no. supplement 1, 2000, page 528) describes that pyrurate protects cerulein induced acute pancreatitis.

WO 03/088955 discloses a pharmaceutical composition comprising an alpha-keto alkanoic acid ester or amine and lactic acid or a lactic acid salt.

### SUMMARY OF THE INVENTION

It has been found that certain α-keto esters and α-keto amides can be used to ameliorate the effects of acute alcoholic hepatitis. For example, when Ringer's Ethyl Pyruvate Solution (REPS) was administered to laboratory C57/BL6 mice after inducing an acute liver injury according to a model of binge drinking, REPS decreased the occurrence of acute alcoholic hepatitis, compared to control mice administered Ringer's Lactate Solution (Examples 2-5). Accordingly, disclosed herein is a method for treating subjects that have or are at risk for developing hepatitis, e.g., hepatitis caused by alcohol and other toxins.

The instant invention relates to a method of treating or ameliorating the effects of hepatitis. Typically, the hepatitis is caused by a toxin such as alcohol, drugs or chemicals. The method comprises administering to the subject an effective amount of an ester of an alpha-ketoalkanoic acid or an amide of an alpha-ketoalkanoic acid. Most commonly, the method is used to treat hepatitis caused by alcohol, hereinafter referred to as "alcoholic hepatitis".

The method of the present invention has several advantages. The therapeutic or prophylactic treatment of acute alcoholic hepatitis using the compounds described herein alleviates the symptoms of acute alcoholic hepatitis. In addition, by treating acute alcoholic hepatitis as described herein, the recovery time for patients with alcoholic hepatitis can be reduced.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

FIG. 1 is a graph showing the effects of Ringer's Lactate Solution (RLS) or Ringer's Ethyl Pyruvate Solution (REPS) on hepatic TNF-α expression in mice with alcoholic hepatitis.

FIG. 2 is a graph showing the effects of Ringer's Lactate Solution (RLS) or Ringer's Ethyl Pyruvate Solution (REPS) on hepatic malondialdehyde content in mice with alcoholic hepatitis.

FIG. 3 is a graph showing the effects of Ringer's Lactate Solution (RLS) or Ringer's Ethyl Pyruvate Solution (REPS) on plasma Alanine Aminotransferase (ALT) concentration in mice with alcoholic hepatitis.

### DETAILED DESCRIPTION OF THE INVENTION

A description of referred embodiments of the invention follows. The present invention relates to a method of treating hepatitis in a subject by administering an ester of an alpha-ketoalkanoic acid or an amide of an alpha-ketoalkanoic acid dissolved in a physiologically-acceptable vehicle. The disclosed method can be used to treat hepatitis caused by alcohol, i.e. alcoholic hepatitis. Alternatively, the disclosed method is used to treat hepatitis caused by poisons, chemical agents and drugs.

"Alcoholic hepatitis" is a precursor to cirrhosis and is caused by alcohol. The typical histologic picture includes hepatocellular necrosis and ballooning degeneration, and alcoholic Mallory's hyaline bodies (abnormal aggregations of cellular intermediate filament proteins indicative of fibrosis). Cholestasis is prominent. Alcoholic hepatitis can range from a mild hepatitis, with abnormal laboratory tests being the only indication of disease, to severe liver dysfunction with complications such as jaundice (yellow skin caused by bilirubin retention), hepatic encephalopathy (neurological dysfunction caused by liver failure), ascites (fluid accumulation in the abdomen), bleeding esophageal varices (varicose veins in the esophagus), abnormal blood clotting and coma. Alcoholic hepatitis is reversible if the patient stops drinking, but it usually takes several months to resolve. Alcoholic hepatitis can lead to liver scarring and cirrhosis. If the liver abnormalities last less than about six months, the disease will be considered acute hepatitis; if the disease course becomes longer than about six months, the hepatitis is considered chronic.

Many drugs and chemical agents can cause liver damage and induce hepatitis, such as amethopterin, tetracycline, acetaminophen, fenoprofen, and the like. The degree and severity of the liver damage is dependent on the dosage, the length of the course, and individual's constitution. Long-term exposure to drugs and/or chemicals can induce chronic hepatitis, and even cirrhosis. The disclosed methods are effective in treating hepatitis caused by these agents. Commonly, there is a period of time between exposure to these agents (or initial exposure) to these agents and the onset of symptoms associated with the hepatitis, e.g., at least one week, one month, two months, six months or a one year.

The disclosed methods are also effective in ameliorating the effects of anti-viral agents such as *interferon* alpha and ribavirin, which can cause or excaberate hepatitis. Co-administration of the compositions described herein with such anti-viral agents is also within the scope of the disclosed invention.

The disclosed methods can also be used prophylactically, i.e., to treat subjects at risk of developing certain types of hepatitis. For example, subjects with mild or chronic alcoholic hepatitis or a subject infected with a viral hepatitis such as hepatitis C are at greater risk for further liver complications when exposed to toxic agents or undergoing treatment with certain drugs, as discussed previously. The disclosed method can be used prophylactically before such exposure or treatment begins. The method of the present invention can also be administered prior to or subsequent to binge drinking to prevent, inhibit or reduce the occurrence of acute alcoholic hepatitis.

In one aspect, the therapeutic agent used in the method disclosed herein is an effective amount of an ester of an alpha-ketoalkanoic acid, for example, a C₃-C₈ straight-chained or branched alpha-ketoalkanoic acid. Examples include alpha-keto-butyrate, alpha-ketopentanoate, alpha-keto-3-methyl-butyrate, alpha-keto-4-methyl-pentanoate or alpha-keto-hexanoate. Pyruvate is preferred. A variety of groups such as alkyl, aralkyl, alkox-yalkyl, carbalkoxyalkyl or acetoxyalkyl are suitable for the ester position of the molecule. Specific examples include ethyl, propyl, butyl, carbmethoxymethyl (-CH₂COOCH₃), carbethoxymethyl (-CH₂COOCH₂CH₃), acetoxymethyl (-CH₂OC(O)CH₃), carbmethoxyethyl (-CH₂CH2COOCH₃), carbethoxyethyl (-CH₂CH₂COOCH₂CH₃), methoxymethyl (-CH₂OCH₃) and ethoxymethyl (-CH₂OCH₂CH₃). Ethyl esters are preferred. Thiolesters (e.g., wherein the thiol portion is cysteine or homocysteine) and glyceryl esters (e.g., wherein one or more of the alcohol groups on glycerol are replaced with an α-ketoalkanoate group) are also included. Other groups suitable for esterification of alpha-ketoalkanoic acids include: 1) dihydroxyacetone esters of formula: 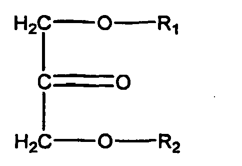 wherein R₁ is an α-ketoalkanoate group such as pyruvyl and R₂ is H, an α-ketoalkanoate group such as pyruvyl or a C1-C3 acyl group such as acetyl or propionyl; and 2) monosaccharide esters such as ribosyl and glucosyl esters: 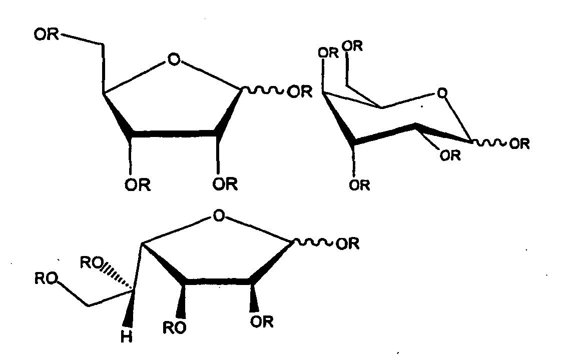 wherein each R is independently H, an α-ketoalkanoate group such as pyruvyl or a C1-C3 acyl group such as acetyl or propionyl, provided that at least one R is an α-ketoalkanoate group.

Specific example of alpha-ketoalkanoic esters suitable for use in the disclosed method include ethyl pyruvate, propyl pyruvate, carbmethoxymethyl pyruvate, acetoxymethyl pyruvate, carbethoxymethymethyl pyruvate, ethoxymethyl pyruvate, ethyl alpha-keto-butyrate, ethyl alpha-keto-pentanoate, ethyl alpha-keto-3-methyl-butyrate, ethyl alpha-keto-4-methyl-pentanoate, or ethyl alpha-keto-hexanoate. Ethyl pyruvate is a preferred alpha-keto-acid ester.

In yet another aspect, the therapeutic agent used in the method disclosed herein is an effective amount of an amide of an alpha-ketoalkanoic acid. Suitable amides of alpha-ketoalkanoic acids for use in the method of the present inventions include compounds having the following structural formula: RCOCONR1R2. R is an alkyl group; R1 and R2 are independently -H, alkyl, aralkyl, alkoxyalkyl, carbalkoxyalkyl or -CHR3COOH (i.e. an "amino acid amide" of an alpha-ketoalkanoic acid); and R3 is the side chain of a naturally occurring amino acid. Preferably, the amide of an alpha-ketoalkanoic acids is a pyruvamide.

Suitable alkyl groups include C₁-C₈ straight chained or branched alkyl group, preferably C₁-C₆ straight chained alkyl groups.

Suitable aryl groups include carbocyclic (e.g., phenyl and naphthyl) and heterocyclic (e.g., furanyl and thiophenyl) aromatic groups, preferably phenyl.

An alkoxy group is -OR4, wherein R4 is an alkyl group, as defined above. An alkoxyalkyl group is an alkyl group substituted with -OR4.

An aralkyl group is -XY, wherein X is an alkyl group and Y is an aryl group, both as defined above.

A carboxyalkyl group is an alkyl group substituted with -COOH. A carbalkoxyalkyl group is an alkyl group substituted with -C(O)OR, wherein R is an alkyl group, as defined above.

An acyl group is -C(O)-R, wherein R is an alkyl group, as defined above.

An acetoxy alkyl group is an alkyl group substituted with -O-C(O)-R, wherein R is an alkyl group, as defined above.

The terms "therapeutic" and "treatment" as used herein, refer to ameliorating symptoms associated with a disease or condition, including preventing, inhibiting or delaying the onset of the disease symptoms, and/or lessening the severity, duration or frequency of symptoms of the disease.

A "subject" is preferably a human patient, but can also be a companion animal (e.g., dog, cat and the like), a farm animal (e.g., horse, cow, sheep, and the like) or laboratory animal (e.g., rat, mouse, guinea pig, and the like).

Formulation of a therapeutic agent to be administered will vary according to the route of administration selected (e.g., solution, emulsion, capsule). An appropriate composition comprising the agent to be administered can be prepared in a physiologically or pharmaceutically acceptable vehicle or carrier. A physiologically or pharmaceutically acceptable carrier for the composition used in the method of the present invention can be any carrier vehicle generally recognized as safe for administering a therapeutic agent to a mammal, e.g., a buffer solution for infusion or bolus injection, a tablet for oral administration or in gel, micelle or liposome form for on-site delivery. A preferred buffer solution is water or isotonic or hypertonic saline buffered with bicarbonate, phosphate, lactate or citrate at 0.1 M to 0.2 M. Alternatively, the therapeutic agent is administered in a plasma extender, microcolloid or microcrystalline solution. One preferred carrier is Ringer's isotonic saline solution comprising from about 105 mM to 110 mM NaCl, from about 3.8 mM to about 4.2 mM KCl, and from about 2.5 to 2.9 mM CaCl₂. More preferably, the carrier is Ringer's Lactate solution comprising from about 105 mM to 110 mM NaCl, from about 3.8 mM to about 4.2 mM KCl, and from about 2.5 to 2.9 mM CaCl₂, and from about 25 mM to about 30 mM of a lactate salt such as sodium lactate. Preferably, acidity of the formulation is adjusted to a pH range of about 4 to about 8, even more preferably to a pH value of about 5 to about 7. Other carriers for the compounds of the present invention include isotonic salt solutions buffered with citrate, for example, approximately 100 mM to 200 mM citrate.

A preferred concentration range of the therapeutic agent is from about 0.1 to about 10% by weight. In a particularly preferred aspect, the pharmaceutical composition comprises approximately 10 mg/ml of ethyl pyruvate. A preferred example of the formulation used for treating alcoholic hepatitis comprises 2% to 3% ethyl pyruvate by weight, approximately 100 mM citrate buffer (or about 25 mM to about 30 mM of sodium lactate), about 4 mM KCl and, optionally, 2.7 mM CaCl₂. The formulation administered for the treatment of acute alcoholic hepatitis can be formed by admixing components of a two part formulation, one part containing, for example, ethyl pyruvate (neat), and the second part consisting of the remaining components of a desired aqueous formulation, for example, those reagents described above.

The pharmaceutical compositions used in the method of the present invention can optionally include an enolization agent when the therapeutic agent is an α-keto ester. The enolization agent and an α-keto ester are contained in a physiologically acceptable carrier. An "enolization agent" is a chemical agent, which induces and stabilizes the enol resonance form of an alpha-keto ester at or around physiological pH (e.g., between about 4.0 to about 8.0, more preferably between about 4.5 to about 6.5). Enolization agents include a cationic material, preferably a divalent cation such as calcium or magnesium or, for example, a cationic amino acid such ornithine or lysine. Divalent cations are introduced into the pharmaceutical formulation as a salt, e.g., as calcium chloride or magnesium chloride. The enolization agent in the composition of the invention is at an appropriate concentration to induce enolization of the alpha-keto functionality of the amount of active ester agent in the administered composition, e.g., from 0.0 to 4.0 molar equivalents relative to the ester.

The precise dose to be employed in the formulation of a therapeutic agent will depend on the route of administration, and the seriousness of the conditions, and should be decided according to the judgment of a practitioner and each patient's circumstances. Effective doses may be extrapolated from dose-response curves derived from *in vitro* or animal model test systems.

According to the method, an ester of an alpha-ketoalkanoic acid or an amide of alpha-ketoalkanoic acid can be administered to a subject by an appropriate route, either alone or in combination with another drug. An effective amount of an ester of an alpha-ketoalkanoic acid or an amide of alpha-ketoalkanoic acid is administered. An effective amount is an amount sufficient to achieve the desired therapeutic or prophylactic effect, under the conditions of administration, such as an amount sufficient for treating (therapeutically or prophylactically), preventing, ameliorating or slowing the onset of the symptoms of alcoholic hepatitis.

The therapeutic compositions of the invention can be administered through a variety of routes, for example, oral, dietary, topical, intravenous, intramuscular, or by inhalation (e.g., intrabronchial, intranasal or oral inhalation, intranasal drops) routes of administration, depending on the agent and disease or condition to be treated, using routine methods in physiologically-acceptable inert carrier substances. Other suitable methods of administration can also include rechargeable or biodegradable devices, and slow release polymeric devices. For example, the therapeutic compositions can be administered in a sustained release formulation using a biodegradable biocompatible polymer, or by on-site delivery using micelles, gels, liposomes, or a buffer solution. Preferably, the pharmaceutical composition is administered as an infusate at a concentration of, e.g., 10 mM to 200 mM, preferably 20 mM to 90 mM of the active agent, at a rate of 1 mg/kg body weight/day to 200 mg/kg body weight/day, in a buffer solution as described herein. More preferably, the pharmaceutical composition is administered as an infusate at a concentration of about 26 to 30 mM of the active agent at a dose of 100 mg/kg body weight/day to 150 mg/kg body weight/day of alpha-ketoalkanoic acid, in a buffer solution. In bolus form, the active agent can be administered at a similar dosage, e.g., 1 mg/kg body weight/day to 200 mg/kg body weight/day of active agent, where the dosage is divided into aliquots and delivered 1 to 4 times daily (for a total dosage of 1 mg/kg body weight/day to 200 mg/kg body weight/day), with the concentration of the active agent adjusted accordingly. Optimal dosage and modes of administration can readily be determined by conventional protocols.

The α-keto amides and α-keto esters disclosed herein for the treatment of hepatitis can be administered as a monotherapy (i.e., alone as the sole therapeutic agents being used to treat the hepatitis) or in combination with other pharmaceutical agents, e.g., agents used in the treatment of hepatitis such as interferon alpha and ribavirin. In addition, the α-keto amides and α-keto esters can be administered in combination with anti-microbials, anti-inflammatory agents, analgesics, anti-viral agents, anti-fungals, anti-histamines and the like.

Examples of suitable anti-microbial agents include sulfa drugs, pencillins (e.g., Benzyl penicillin, P-hydroxybenzyl penicillin, 2-pentenyl penicillin, *N*-heptyl penicillin, phenoxymethyl penicillin, Phenethicillin, Methicillin, Oxacillin, Cloxacillin, Dicloxacillin, Flucloxacillino, Nafcillin, Ampicillin, Amoxicillin, Cyclacillin, Carbenicillin, Ticarcillin, Piperacillin, Azlocillin, Meczlocillin, Mecillinam, Amdinocillin), Cephalosporin and derivatives thereof (e.g, Cephalothin, Cephapirin, Cephacetrile, Cephazolin, Caphalexin, Cephandine, Cefadroxil, Cefamandol, Cefuroxime, Ceforanide, Cefoxitin, Cefotetan, Cefaclor, Cefotaxime, Ceftizoxime, Ceftrioxone, Ceft*azidime, Moxalactam, Cefoperazone, Cefixime, Ceftibuten and Cefprozil), Oxolinic Acid, Amifloxacin, Temafloxacin, Nalidixic Acid, Piromidic Acid, Ciprofloxacin, Cinoxacin, Norfloxacin, Perfloxacin, Rosaxacin, Ofloxacin, Enoxacin, Pipemidic Acid, Sulbactam, Clavulinic Acid, 6-Bromopenicillanic Acid, β-Chloropenicillanic Acid,6-Acetylmethylene-Penicillanic Acid, Cephoxazole, Sultampicillin, Formaldehyde Hudrate Ester of Adinocillin and Sulbactam, Tazobactam, Aztreonam, Sulfazethin, Isosulfazethin, Norcardicins, *m-*Carboxyphenyl Phenylacetamidomethylphosphonate, Chlortetracycline, Oxytetracyline, Tetracycline, Demeclocycline, Doxycycline, Methacycline and Minocycline.

Examples of suitable anti-inflammatory agents include examples of suitable NSAIDs include aminoarylcarboxylic acid derivatives (e.g., Enfenamic Acid, Etofenamate, Flufenamic Acid, Isonixin, Meclofenamic Acid, Niflumic Acid, Talniflumate, Terofenamate and Tolfenamic Acid), arylacetic acid derivatives (e.g., Acematicin, Alclofenac, Amfenac, Bufexamac, Caprofen, Cinmetacin, Clopirac, Diclofenac, Diclofenac Sodium, Etodolac, Felbinac, Fenclofenac, Fenclorac, Fenclozic Acid, Fenoprofen, Fentiazac, Flubiprofen, Glucametacin, Ibufenac, Ibuprofen, Indomethacin, Isofezolac, Isoxepac, Ketoprofen, Lonazolac, Metiazinic Acid, Naproxen, Oxametacine, Proglumrtacin, Sulindac, Tenidap, Tiramide, Tolectin, Tolmetin, Zomax and Zomepirac), arylbutyric acid ferivatives (e.g., Bumadizon, Butibufen, Fenbufen and Xenbucin) arylcarboxylic acids (e.g., Clidanac, Ketorolac and Tinoridine), arylproprionic acid derivatives (e.g., Alminoprofen, Benoxaprofen, Bucloxic Acid, Carprofen, Fenoprofen, Flunoxaprofen, Flurbiprofen, Ibuprofen, Ibuproxam, Indoprofen, Ketoprofen, Loxoprofen, Miroprofen, Naproxen, Oxaprozin, Piketoprofen, Piroprofen, Pranoprofen, Protinizinic Acid, Suprofen and Tiaprofenic Acid), pyrazoles (e.g., Difenamizole and Epirizole), pyrazolones (e.g., Apazone, Benzpiperylon, Feprazone, Mofebutazone, Morazone, Oxyphenbutazone, Phenylbutazone, Pipebuzone, Propyphenazone, Ramifenazone, Suxibuzone and Thiazolinobutazone), salicyclic acid derivatives (e.g., Acetaminosalol, 5-Aminosalicylic Acid, Aspirin, Benorylate, Biphenyl Aspirin, Bromosaligenin, Calcium Acetylsalicylate, Diflunisal, Etersalate, Fendosal, Flufenisal, Gentisic Acid, Glycol Salicylate, Imidazole Salicylate, Lysine Acetylsalicylate, Mesalamine, Morpholine Salicylate, 1-Naphthyl Sallicylate, Olsalazine, Parsalmide, Phenyl Acetylsalicylate, Phenyl Salicylate, 2-Phosphonoxybenzoic Acid, Salacetamide, Salicylamide *O*-Acetic Acid, Salicylic Acid, Salicyloyl Salicylic Acid, Salicylsulfuric Acid, Salsalate and Sulfasalazine), thiazinecarboxamides (e.g., Droxicam, Isoxicam, Piroxicam and Tenoxicam), α-Acetamidocaproic Acid, *S*-Adenosylmethionine, 3-Amino-4-hydroxybutyric Acid, Amixetrine, Bendazac, Benzydamine, Bucolome, Difenpiramide, Ditazol, Emorfazone, Guaiazulene, Ketorolac, Meclofenamic Acid, Mefenamic Acid, Nabumetone, Nimesulide, Orgotein, Oxaceprol, Paranyline, Perisoxal, Pifoxime, Piroxicam, Proquazone and Tenidap.

Examples of suitable analgesics include an opioid (e.g. morphine), a COX-2 inhibitor (e.g., Rofecoxib, Valdecoxib and Celecoxib), salicylates (e.g., ASPIRIN, choline magnesium trisalicylate, salsalate, difunisal and sodium salicylate), propionic acid derivatives (e.g., fenoprofen calcium, ibuprofen, ketoprofen, naproxen and naproxen sodium, indoleacetic acid derivatives (e.g., indomethacin, sulfindac, etodalac and tolmetin), fenamates (e.g., mefenamic acid and meclofenamate), benzothiazine derivatives or oxicams (e.g., mobic or piroxicam) or pyrrolacetic acid (e.g., ketorolac).

Examples of suitable anti-viral agents include inferno gamma, ribavirin, fialuridine, acyclovir, ganciclovir, penciclovir, famciclovir, PMEA, bis-POM PMEA, lamivudine, cytallene, oxetanocins, carbocyclic oxetaoncins, foscarnet, phyllanthus amarus, N-acety-L-cysteine, destruxin B, hypericin, aucubin and N-butyldeoxynojirimycin.

Examples of suitable anti-fungals include amphotericin B, nystatin, itraconazole, fluconazole, ketoconazole, miconazole, flucytosine and dapsone.

The present invention will now be illustrated by the following Examples.

### Example 1 Induction of Acute Liver Injury by a Binge Drinking Mouse Model

All animals were fed standard laboratory chow and allowed to acclimatize for 7 days. After acclimation, acute liver injury was induced using a model of binge drinking originally described by Carson EJ, Pruett SB. "Development and characterization of a binge drinking model in mice for evaluation of the immunological effects of ethanol", Alcohol Clin. Exp. Res.20: 132-138 (1996) and subsequently modified by Zhou Z, et al. "Metallothionein protection against alcoholic liver injury through inhibition of oxidative stress" Exp. Biol. Med. 227: 214-222 (2002) and Zhou Z., et al., "Metallothionein-independent zinc protection from alcoholic liver injury", Am. J. Pathol. 160: 2267-2274 (2002), the entire teachings of which are incorporated herein by reference. This model was designed to achieve blood alcohol levels, behavioral effects, and physiological changes comparable with human binge drinking. Mice were divided into three groups (n=8-10 each). Mice in the Ringer's lactate solution (RLS) group received ethanol (5 g/kg body weight) every 12 hours for a total of three doses. The ethanol was administered by gavage as a 25% (w/v) aqueous solution. Beginning one hour after the last dose of ethanol, the mice were injected intraperitoneally (i.p.) with 0.4 ml of RLS every six hours for a total of three doses. Mice in the Ringer's ethyl pyruvate solution (REPS) group were dosed with alcohol according the same schedule as the animals in the RLS group. Subsequently, however, instead of being treated with RLS, these mice received three i.p. injections of REPS, which was formulated as previously described (Yang R, et al., "Ethyl pyruvate modulates inflammatory gene expression in mice subjected to hemorrhagic shock", Am. J. Physiol. Gastrointest. Liver Physiol. 283:G212-G22 (2002), incorporated herein by reference in its entirety). Each dose of REPS provided 40 mg/kg of EP in 0.4 ml of a solution that also contained 130 mM NaCl, 4 mM KCl and 2.7 mM CaCl₂. REPS was injected every 6 hours beginning one h after administration of the last dose of ethanol. Mice in the control (CONT) group were not gavaged with ethanol but rather received an equal volume of an isocaloric solution of maltose. Mice in this group were not treated with either RLS or REPS. Nineteen hours after the last dose of ethanol or maltose, all of the mice were anesthetized with pentobarbital (90 mg/kg i.p.), and the following procedures were performed: a segment of ileum was harvested for determination of mucosal permeability; the mesenteric lymph node (MLN) complex was harvested to measure bacterial translocation; blood was aspirated from the heart to measure the plasma concentration of alanine aminotransferase (ALT); and a portion of the liver was removed for determination of NF-kB activation using the electrophoretic mobility shift assay (EMSA), expression of TNF mRNA using semi-quantitative RT-PCR, and histopathology. The hepatic tissue to be used for EMSA or RT-PCR was immediately frozen at -80°C, whereas the tissue for histopathology was immediately fixed in 10% formalin.

### Example 2 REPS Inhibits Inflammatory Response in a Mouse Modal of Alcoholic Hepatitis as Measured by TNF-α Activation

Increased hepatic TNF-α expression has been implicated in the pathogenesis of early alcohol-induced liver injury in mice. (Yin M. et al., "Essential role of tumor necrosis factor alpha in alcohol-induced liver injury in mice", Gastroenterology 117: 942-952 (1999)).

Following the induction of acute hepatitis as described in Example 1, total RNA was extracted from harvested hepatic tissue samples with chloroform and TRI Reagent (Molecular Research Center, Cincinnati, OH) as directed by the manufacturer. The total RNA was treated with DNAFree® (Ambion, Houston, TX) as instructed by the manufacturer using 10 units of DNase 1/10 µg RNA. Two µg of total RNA was reverse transcribed in a 40 µl reaction volume containing 0.5 µg of oligo(dT)₁₅ (Promega), 1 mM of each dNTP, 15 U AMV reverse transcriptase (Promega), and 1 U/µL of recombinant RNasin ribonuclease inhibitor (Promega) in 5 mM MgCl₂, 10 mM Tris-HCl, 50 mM KCL, Q.1 % Triton X-100 (pH=8.0). The reaction mixtures were preincubated at 21°C for 10 min prior to DNA synthesis. The Reverse Transcriptase (RT) reactions were carried out for 50 min at 42 °C and were heated to 95 °C for 5 min to terminate the reaction. Reaction mixtures (50 µL) for PCR were assembled using 5 µL of cDNA template, 10 units AdvanTaq Plus DNA Polymerase (Clontech, Palo Alto, CA), 200 µM of each dNTP, 1.5 mM MgCl₂ and 1.0 µM of each primer in 1 × AdvanTaq Plus^{™} PCR buffer. PCR reactions were performed using a Model 480 thermocycler (Perkin Elmer, Norwalk, CT). Amplication of cDNA was initiated with 5 min of denaturation at 94°C. The PCR conditions for amplifying cDNA for TNF and IL-6 were as follows: denaturation at 94°C for 45 s, annealing at 61°C for 45s, and polymerization at 72°C for 45 s. Amplification of cDNA for iNOS was carried out by denaturing at 94 °C for 45 s, annealing at 58 °C for 1 min, and polymerizing at 72 °C for 45 s. To ensure that amplification was in the linear range, we empirically determined that 25, 22; 20 and 33 were the optimal number of cycles for TNF and IL-6 cDNA prepared from RAW 264.7 cell extracts, iNOS cDNA prepared from RAW 264.7 cell extracts, and TNF cDNA prepared hepatic tissue extracts, respectively. After the last cycle of amplification, the samples were incubated at 72 °C for 10 min and then held at 4 °C. The 5' and 3' primers for iNOS were CAC CAC AAG GCC ACA TCG GAT T (SEQ ID NO: 1) and CCG ACC TGA TGT TGC CAT TGT T (SEQ ID NO: 2), respectively (Invitrogen, Carlsbad, CA); the expected product length was 426 bp. The 5' and 3' primers for TNF were GGC AGG TCT ACT TTG GAG TCA TTG C (SEQ ID NO: 3) and ACA TTC GAG GCT CCA GTG AAT TCG G (SEQ ID NO: 4), respectively; the expected product length was 307 bp. The 5' and 3' primers for IL-6 were TTC CAT CCA GTT GCC TTC TTG G (SEQ ID NO: 5) and TTC TCA TTT CCA CGA TTT CCC AG (SEQ ID NO: 6), respectively; the expected product length was 174 bp. 18S ribosomal RNA was amplified to verify equal loading. For this reaction, the 5' and 3' primers were CCC GGG GAG GTA GTG ACG AAA AAT (SEQ ID NO: 7) and CGC CCG CTC CCA AGA TCC AAC TAC (SEQ ID NO: 8), respectively; the expected product length was 209 bp. Ten microliters of each PCR reaction were electrophoresed on a 2% agarose gel, scanned at a NucleoVision imaging workstation (NucleoTech, San Mateo, CA), and quantified using GelExpert release 3.5.

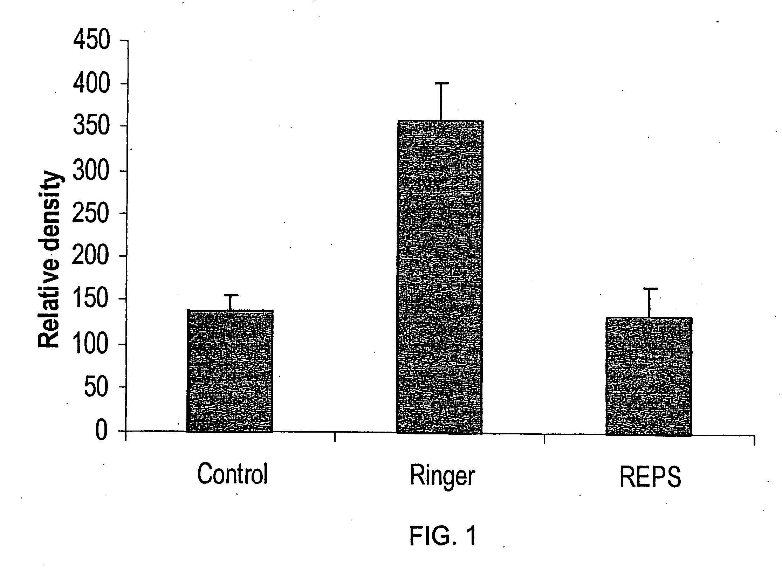

As presented on FIG.1, semi-quantitative RT-PCR showed that hepatic TNF-α mRNA expression was markedly increased when mice were treated with RLS 1 h after the last of three 5 g/kg doses of alcohol administered enterally over a 12 h period. If the mice were treated with REPS instead of RLS, upregulation of hepatic TNF-α mRNA expression amplification was not observed.

### Example 3 REPS Inhibits Inflammatory Response in a Mouse Model of Alcoholic Hepatitis as Measured by NF-kB Activation

Acute or subacute administration of ethanol is known to promote hepatic activation of the pro-inflammatory transcription factor, NF-kB. (Nanji A.A. et al. "Curcumin prevents alcohol-induced liver disease in rats by inhibiting the expression of NF-kappaB dependent genes", Am. J. Physiol. Gastrointest. Liver Physiol." (2002); Kono H. et al., "Diphenyleneiodonium sulfate, an NADPH oxidase inhibitor, prevents early alcohol-induced liver injury in the rat", Am. J. Physiol. Gastrointest. Liver Physiol. 280: G1005-G1012 (2002); Spitzer J.A. et al. "Ethanol and LPS modulate NF-kappaB activation, inducible NO synthase and COX-2 gene expression in rat liver cells in vivo", Front. Biosci.7: a99-a108 (2002).) Accordingly, EMSA was used to determine whether treatment with REPS would modulate NF-κB activation in our murine model of binge alcohol consumption.

Following the induction of acute hepatitis as described in Example 1, nuclear extracts were prepared, hepatic tissue samples were homogenized with T-PER^{™} (Pierce, Rockford, IL), using a 1:20 ratio of tissue to the sample preparation reagent, as directed by the manufacturer's instructions. The samples were centrifuged at 10,000 g for 5 min to pellet tissue debris. The supernatant was collected and frozen at -80 °C. Nuclear protein concentration was determined using a commercially available Bradford assay (Bio-Rad, Hercules, CA).

The EMSA for NF-kB nuclear binding was performed using a duplex oligonucleotide probe based on the NF-kB binding site upstream of the murine iNOS promoter as previously described (Yang R, et al., "Ethyl pyruvate modulates inflammatory gene expression in mice subjected to hemorrhagic shock", Am. J. Physiol. Gastrointest. Liver Physiol. 283: G212-G22 (2002)). The sequence of the double-stranded NF-kB oligonucleotide was as follows: sense: 5'-AGT TGA GGG GAC TTT CCC AGG C-3' (SEQ ID NO: 9); antisense: 3'-TCA ACT CCC CTG AAA GGG TCC G-5' (SEQ ID NO: 10) (Promega; Madison, WI). The oligonucleotides were end-labeled with γ-³²P adenosine triphosphate (New England Nuclear; Boston, MA) using T4 polynucleotide kinase (Promega; Madison, WI). 6 µg of nuclear protein was incubated at room temperature with γ-³²P -labeled NF-kB probe in 4 µl of 5X binding buffer (65 mM HEPES, 325 mM NaCl, 5 mM DTT, 0.7 mM EDTA, 40% glycerol, pH=8.0) in the presence of 2 µg of polyDI-DC for 20 min, the total volume of the binding reaction mixture being 20 µL. The binding reaction mixture was electrophoresed on 4% nondenaturing polyacrylamide electrophoresis gels. After electrophoresis, the gels were dried and exposed to Kodak (Rochester, NY) X-Omat film at -80 °C. The specificity of the binding reaction has been previously verified by carrying out appropriate cold-competition and super-shift assays.

As assessed by gel electrophoresis, there was a marked increase in activation of NF-xB following the induction of hepatitis in RLS-treated mice. However, treatment of mice with REPS after alcohol ingestion down-regulated NF-κB-DNA binding.

### Example 4 REPS Prevents Lipid Peroxidation as Measured in a Mouse Model of Alcoholic Hepatitis by Formation of Malondialdehyde, a Marker of Redox Stress

Acute alcohol intoxication has been associated with lipid peroxidation in both humans and rodents. Furthermore, in rats subjected to hemorrhagic shock and resuscitation, treatment with EP decreases hepatic lipid peroxidation. Accordingly, we sought to determine whether a similar beneficial effect of EP treatment would be observed in our murine model of binge drinking.

Following the induction of acute hepatitis as described in Example 1, the assay for lipid peroxidation was performed as described in Tawadrous Z.S., et al. "Resuscitation from hemorrhagic shock with Ringer's ethyl pyruvate solution improves survival and ameliorates intestinal mucosal hyperpermeability in rats", Shock 17: 473-477 (2002), incorporated herein by reference in its entirety. Briefly, after thawing the tissue specimens, 2 ml of phosphate buffer (0.05 M, pH=7.4) was added to 1.0 g of tissue. The tissue was homogenized. Trichloroacetic acid (20% v/v solution; 2.5 ml) and thiobarbituric acid (0.67% w/v solution; 1.0 ml) were added to 0.5 ml of the tissue homogenate. The color of thiobarbituric acid pigment was developed by incubating the mixture in a 100 °C water bath for 30 min. After cooling the mixture to room temperature by immersion in tap water, 4 ml of n-butanol was added and shaken vigorously. After centrifugation, absorbance of the butanol layer was determined at 535 nm. Samples were run in duplicate and the results were averaged. 1,1,3,3-tetrathoxypropane was used to generate a standard curve. Results were expressed as nanomoles of malondialdehyde (MDA) per gram of tissue.

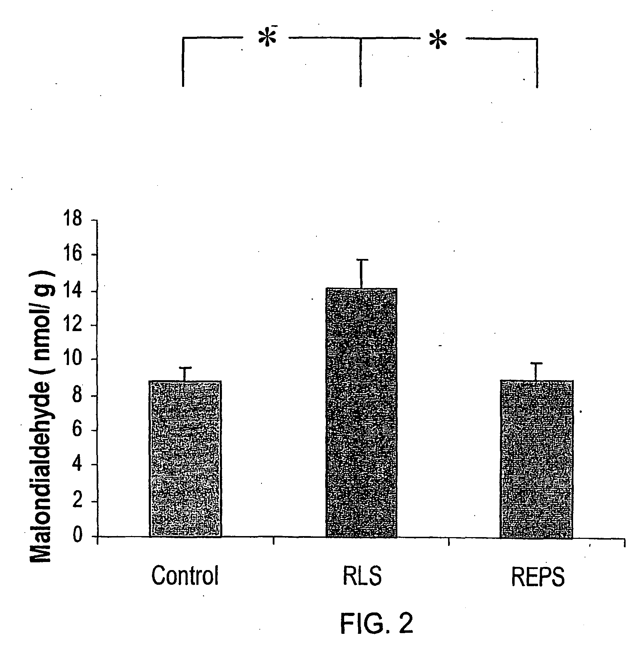

The mean tissue MDA concentration was significantly greater in the RLS group than in the control group (FIG. 2). However, the mean level of this marker of liver lipid peroxidation was significantly lower in the REPS group than in the RLS group, indicating amelioration of liver damage in REPS-treated mice.

### Example 5 Evidence of Induction of Hepatocellular Damage in a Mouse Model of Alcoholic Hepatitis as Measured by Assessing Plasma Alanine Amino Transferase

200 µL of blood was obtained by cardiac puncture and placed in a 0.5 ml centrifugation tube on ice. The samples were centrifuged at 5,000 g for 3 min. The serum was collected and assayed for Alanine Amino Transferase (ALT) using an automated assay system.

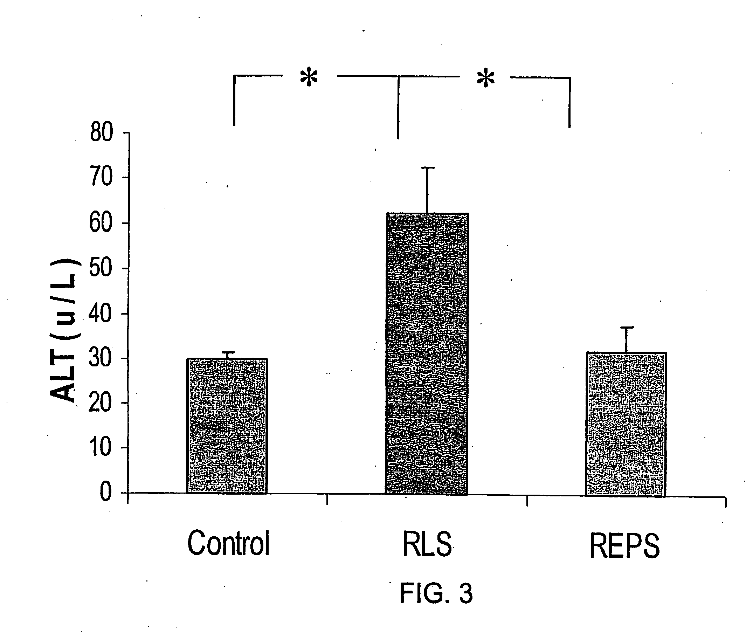

As shown in FIG. 3, the mean plasma ALT concentration 19 hours after the last dose of alcohol or maltose solution was significantly greater in the RLS group than in the control (CONT) group. However, the mean circulating level of this biochemical marker of hepatocellular injury was significantly lower in the REPS group than in the RLS group.

### Example 6 Treatment with PERS Prevents Hepatocellular Damage in a Mouse Model of Alcoholic Hepatitis as Evidenced by Histological Examination

Forrnalin-fixed hepatic tissue was sectioned, stained with hematoxylin and eosin, and examined using light microscopy at 600x and 1000x magnification.

The examination showed that in the RLS group, hepatic sections revealed extensive evidence of fatty change in the portal areas and parts of the lobules. At high magnification, globular red hyaline material was evident within hepatocytes and scattered piecemeal necrosis of hepatocytes was apparent. In contrast, in the REPS group fatty changes and necrosis in lobules were reduced considerably and hyaline material was not evident.

Results in Examples 2-5 are presented as means ± SEM. Differences in CFU between groups were analyzed using Wilcoxen's U-test. Other continuous data were analyzed using student's t-test or analysis of variance followed by Fisher's LSD test, as appropriate. P values < 0.05 were considered significant. Summary statistics are presented for densitometry results from studies using RT-PCR to estimate iNOS, TNF-α and IL-6 mRNA expression, but these results were not subjected to statistical analysis since the method employed was only semiquantitative and the samples sizes (n=3-4) were small. (Ulloa L, et al. "Ethyl pyruvate prevents lethality in mice with established lethal sepsis and systemic inflammation", Proc. Natl. Acad. Sci. USA 99: 12351-12356 (2002); Sappington P.L., et al. "Ethyl pyruvate ameliorates intestinal epithelial barrier dysfunction in endotoxemic mice and immunostimulated Caco-2 enterocytic monolayers", J. Pharmacol. Exp. Ther. 304: 464-476 (2003); Yang R., et al. "Ethyl pyruvate modulates inflammatory gene expression in mice subjected to hemorrhagic shock", Am. J. Physiol. Gastrointest. Liver Physiol. 283:G212-G22 (2002).)

## Claims

1. Use of an ester of an alpha-ketoalkanoic acid or an amide of an alpha-ketoalkanoic acid in the manufacture of a medicament for treating hepatitis in a subject,

2. The use of Claim 1 wherein the hepatitis is acute alcoholic hepatitis.

3. The use of Claim 1 wherein the hepatitis is chronic alcoholic hepatitis.

4. The use of Claim 1 wherein the hepatitis is caused by a chemical agent or drug.

5. The use of Claim 1 wherein said medicament is for treating hepatitis prophylactically.

6. The use of Claim 1, wherein an ester of alpba-ketoalkanoic acid is used and wherein said ester of an alpha-ketoalkanoic acid is a C₃-C₈ straight-chainsd or branched alpha-ketoalkanoic acid ester.

7. The use of Claim 6, wherein said ester of an alpha-ketoalbmoic acid is an alkyl, aralkyl, alkoxyalkyl, carbalkoxyalkyl or acetoxyalkyl ester.

8. The use of Claim 6, wherein said ester of an alpha-ketoallcanoic acid is selected from the group consisting of ethyl pyruvate, propyl pyruvate, carboxymethyl pyruvate, acetoxymethyl pyruvate, carbethoxymethymethyl pyruvate, and ethoxymethyl pyruvate.

9. The use of Claim 6, wherein said ester of an alpha-ketoalkanoic acid is selected from the group consisting of ethyl alpha-keto-butyrate, ethyl alpha-keto-pentanoate, ethyl alpha-keto-3-methyl-butyrate, ethyl alpha-keto-4-methyl-pentanoate, and ethyl alpha-keto-hexanoate.

10. The use of Claim 6, wherein said ester of an alpba-ketoalkanoic acid is an ethyl ester.

11. The use of Claim 6, wherein said ester of an alpba-ketoalkanoic acid is an ester of pyruvic acid.

12. The use of Claim 6, wherein said ester of an alpha-ketoalkanoic acid is ethyl pyruvate.

13. The use of Claim 6, wherein said ester of as alpha-ketoalkanoic acid is contained in Ringer's isotonic saline.

14. The use of Claim 6, wherein said ester of an alpha-ketoalkanoic acid is contained in a physiologically-acceptable carrier, which additionally comprises lactate.

15. The use of Claim 14, wherein said physiologically-acceptable carrier further comprises a physiologically-acceptable enolization agent.

16. The use of Claim 14, wherein said physiologically-acceptable carrier is Ringer's isotonic saline comprising potassium ion and/or sodium ion.

17. The use of Claim 6, wherein said alpha-ketoalkanoic acid ester is a glyceryl ester.

18. The use of Claim 6, wherein said alpha-ketoalkanoic acid ester is a ribosyl ester represented by the following formula: 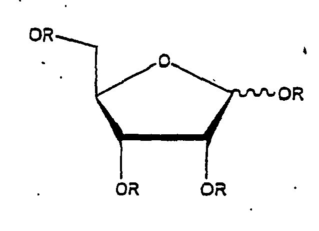 wherein each R is independently H, an alpha-ketoalkanoate group or a C1-C3 acyl and at least one R is an alpha-ketoalkanoate group.

19. The use of Claim 6, wherein said alpha-ketoalkanoic acid ester is a glucosyl ester described by formulae (I) or (II): 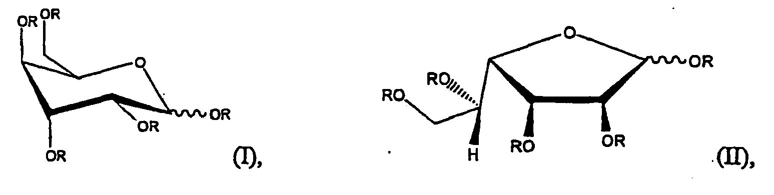 wherein each R is independently H, an alpha-ketoalkanoate group or a C1-C3 acyl and at least one R is an alpha-ketoallcanoate group.

20. The use of Claim 6, wherein said alpha-ketoalkanoic acid ester is a dihydroxyacetone ester.

21. The use of Claim 6, wherein said alpha-ketoalkanoic acid ester is a thiolester.

22. The use of Claim 21, wherein a thiol portion of said thiolester is cysteine or homocysteine.

23. The use of Claim 1 wherein an amide of an alpha-ketoalkanoic acid is used

24. The use of Claim 23, wherein said amide of an alpha-ketoalkanoic acid is a pyruvamide.

25. The use of Claim 23, wherein said amide is an amino acid amide of an alpha-ketoalkanoic acid.

26. Use of ethyl pyruvate in the manufacture of a medicament for treating alcoholic hepatitis.

27. The use of Claim 26 wherein alcoholic hepatitis is acute alcoholic hepatitis.

28. The use of Claim 26 wherein alcoholic hepatitis is chronic alcoholic hepatitis.

29. An ester of an alpha-ketoalkanoic acid or an amide of an alpha-katoalkanoic acid for use in the treatment of hepatitis in a subject.

30. An ester according to Claim 29, wherein an ester of an alpha-ketoalkanoic acid is used and said ester is ethyl pyruvate.

31. An ester according to Claim 29, wherein an ester of a C₃-C₈ straight chained or branched alpha-ketoalkanolc acid is used and said ester is contained in Ringer's isotonic saline.

32. Ethyl pyruvate for use in the treatment of alcoholic hepatitis.

## Patentansprüche

1. Verwendung eines Esters einer alpha-Ketoalkansäure oder eines Amids einer alpha-Ketoalkansäure bei der Herstellung eines Medikaments zur Behandlung von Hepatitis bei einem Subjekt.

2. Verwendung nach Anspruch 1, wobei die Hepatitis akute alkoholische Hepatitis ist.

3. Verwendung nach Anspruch 1, wobei die Hepatitis chronische alkoholische Hepatitis ist.

4. Verwendung nach Anspruch 1, wobei die Hepatitis durch eine chemische Substanz oder Droge verursacht ist.

5. Verwendung nach Anspruch 1, wobei das Medikament zur prophylaktischen Behandlung von Hepatitis vorgesehen ist.

6. Verwendung nach Anspruch 1, wobei ein Ester von alpha-Ketoalkansäure verwendet wird und wobei der Ester einer alpha-Ketoalkansäure ein geradkettiger oder verzweigter C₃-C₈-alpha-Ketoalkansäureester ist.

7. Verwendung nach Anspruch 6, wobei der Ester einer alpha-Ketoalkansäure ein Alkyl-, Aralkyl-, Alkoxyalkyl-, Karbalkoxyalkyl- oder Azetoxyalkylester ist.

8. Verwendung nach Anspruch 6, wobei der Ester einer alpha-Ketoalkansäure aus der Gruppe ausgewählt ist, die besteht aus Ethylpyruvat, Propylpyruvat, Karboxymethylpyruvat, Azetoxymethylpyruvat, Karbethoxymethylmethylpyruvat und Ethoxymethylpyruvat.

9. Verwendung nach Anspruch 6, wobei der Ester einer alpha-Ketoalkansäure aus der Gruppe ausgewählt ist, die besteht aus Ethyl-alpha-ketobutyrat, Ethyl-alpha-ketopentanoat, Ethyl-alpha-keto-3-methylbutyrat, Ethyl-alpha-keto-4-methylpentanoat und Ethyl-alphaketohexanoat.

10. Verwendung nach Anspruch 6, wobei der Ester einer alpha-Ketoalkansäure ein Ethylester ist.

11. Verwendung nach Anspruch 6, wobei der Ester einer alpha-Ketoalkansäure ein Ester von Pyruvinsäure ist.

12. Verwendung nach Anspruch 6, wobei der Ester einer alpha-Ketoalkansäure Ethylpyruvat ist.

13. Verwendung nach Anspruch 6, wobei der Ester einer alpha-Ketoalkansäure in isotonischer Ringer-Salzlösung enthalten ist.

14. Verwendung nach Anspruch 6, wobei der Ester einer alpha-Ketoalkansäure in einem physiologisch akzeptablen Träger enthalten ist, der zusätzlich Laktat aufweist.

15. Verwendung nach Anspruch 14, wobei der physiologisch akzeptable Träger weiterhin ein physiologisch akzeptables Enolisierungsmittel aufweist.

16. Verwendung nach Anspruch 14, wobei der physiologisch akzeptable Träger eine isotonische Ringer-Salzlösung ist, die Kaliumionen und/oder Natriumionen aufweist.

17. Verwendung nach Anspruch 6, wobei der alpha-Ketoalkansäureester ein Glyzerinester ist.

18. Verwendung nach Anspruch 6, wobei der alpha-Ketoalkansäureester ein Ribosylester ist, der durch die folgende Formel wiedergegeben wird: 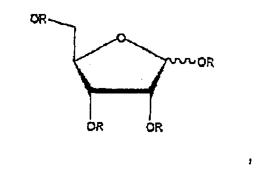 wobei jedes R unabhängig voneinander H, eine alpha-Ketoalkanoatgruppe oder ein C1-C3-Azyl ist und mindestens ein R eine alpha-Ketoalkanoatgruppe ist.

19. Verwendung nach Anspruch 6, wobei der alpha-Ketoalkansäureester ein Glukosylester ist, der durch die Formel (I) oder (11) beschrieben wird: 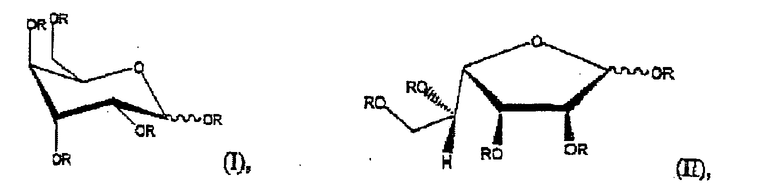 wobei jedes R unabhängig voneinander H, eine alpha-Ketoalkanoatgruppe oder ein C1-C3-Alkyl ist und mindestens ein R eine alpha-Ketoalkanoatgruppe ist.

20. Verwendung nach Anspruch 6, wobei der alpha-Ketoalkansäureester ein Dihydroxyazetonester ist.

21. Verwendung nach Anspruch 6, wobei der alpha-Ketoalkansäureester ein Thiolester ist.

22. Verwendung nach Anspruch 21, wobei ein Thiolanteil des Thiolesters ein Zystein oder Homozystein ist.

23. Verwendung nach Anspruch 1, wobei ein Amid einer alpha-Ketoalkansäure verwendet wird.

24. Verwendung nach Anspruch 23, wobei das Amid einer alpha-Ketoalkansäure in Pyruvamid ist.

25. Verwendung nach Anspruch 23, wobei das Amid ein Aminosäureamid einer alpha-Ketoalkansäure ist.

26. Verwendung von Ethylpyruvat bei der Herstellung eines Medikaments zur Behandlung alkoholischer Hepatitis.

27. Verwendung nach Anspruch 26, wobei alkoholische Hepatitis akute alkoholische Hepatitis ist.

28. Verwendung nach Anspruch 26, wobei alkoholische Hepatitis chronische alkoholische Hepatitis ist.

29. Ester einer alpha-Ketoalkansäure oder eines Amids einer alpha-Ketoalkansäure zur Verwendung bei der Behandlung von Hepatitis bei einem Subjekt.

30. Ester nach Anspruch 29, wobei ein Ester einer alpha-Ketoalkansäure verwendet wird und der Ester Ethylpyruvat ist.

31. Ester nach Anspruch 29, wobei ein Ester einer geradkettigen oder verzweigten C₃-C₈-alpha-Ketoalkansäure verwendet wird und der Ester in isotonischer Ringer-Salzlösung enthalten ist.

32. Ethylpyruvat zur Verwendung bei der Behandlung von alkoholischer Hepatitis.

## Revendications

1. Utilisation d'un ester d'un acide alpha-cétoalcanoïque ou d'un amide d'un acide alpha-cétoalcanoïque pour la fabrication d'un médicament pour traiter une hépatite chez un sujet.

2. Utilisation selon la revendication 1, dans laquelle l'hépatite est une hépatite alcoolique aiguë.

3. Utilisation selon la revendication 1, dans laquelle l'hépatite est une hépatite alcoolique chronique.

4. Utilisation selon la revendication 1, dans laquelle l'hépatite est provoquée par un agent chimique ou une substance médicamenteuse.

5. Utilisation selon la revendication 1, dans laquelle ledit médicament est pour traiter une hépatite de façon prophylactique.

6. Utilisation selon la revendication 1, dans laquelle un ester d'un acide alpha-cétoalcanoïque est utilisé, et dans laquelle ledit ester d'un acide alpha-cétoalcanoïque est un ester d'un acide alpha-cétoalcanoïque en C₃-C₈ à chaîne linéaire ou ramifié.

7. Utilisation selon la revendication 6, dans laquelle ledit ester d'un acide alpha-cétoalcanoïque est un ester alkylique, aralkylique, alcoxyalkylique, carbalcoxyalkylique ou acétoxyalkylique.

8. Utilisation selon la revendication 6, dans laquelle ledit ester d'un acide alpha-cétoalcanoïque est choisi dans le groupe constitué par le pyruvate d'éthyle, le pyruvate de propyle, le pyruvate de carboxyméthyle, le pyruvate d'acétoxyméthyle, le pyruvate de carbéthoxyméthylméthyle et le pyruvate d'éthoxyméthyle.

9. Utilisation selon la revendication 6, dans laquelle ledit ester d'un acide alpha-cétoalcanoïque est choisi dans le groupe constitué par l'alpha-céto-butyrate d'éthyle, l'alpha-céto-pentanoate d'éthyle, l'alpha-céto-3-méthyl-butyrate d'éthyle, l'alpha-céto-4-méthyl-pentanoate d'éthyle et l'alpha-céto-hexanoate d'éthyle.

10. Utilisation selon la revendication 6, dans laquelle ledit ester d'un acide alpha-cétoalcanoïque est un ester éthylique.

11. Utilisation selon la revendication 6, dans laquelle ledit ester d'un acide alpha-cétoalcanoïque est un ester d'acide pyruvique.

12. Utilisation selon la revendication 6, dans laquelle ledit ester d'un acide alpha-cétoalcanoïque est le pyruvate d'éthyle.

13. Utilisation selon la revendication 6, dans laquelle ledit ester d'un acide alpha-cétoalcanoïque est contenu dans une solution saline isotonique de Ringer.

14. Utilisation selon la revendication 6, dans laquelle ledit ester d'un acide alpha-cétoalcanoïque est contenu dans un véhicule physiologiquement acceptable, qui comprend de plus un lactate.

15. Utilisation selon la revendication 14, dans laquelle ledit véhicule physiologiquement acceptable comprend en outre un agent d'énolisation physiologiquement acceptable.

16. Utilisation selon la revendication 14, dans laquelle ledit véhicule physiologiquement acceptable est une solution saline isotonique de Ringer comprenant un ion potassium et/ou un ion sodium.

17. Utilisation selon la revendication 6, dans laquelle ledit ester d'acide alpha-cétoalcanoïque est un ester de glycéryle.

18. Utilisation selon la revendication 6, dans laquelle ledit ester d'acide alpha-cétoalcanoïque est un ester de ribosyle représenté par la formule suivante : 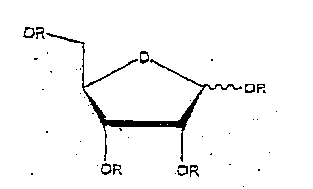 dans laquelle chaque R est indépendamment H, un groupe alpha-cétoalcanoate ou un acyle en C₁-C₃ et au moins un R est un groupe alpha-cétoalcanoate.

19. Utilisation selon la revendication 6, dans laquelle ledit ester d'acide alpha-cétoalcanoïque est un ester de glucosyle représenté par les formules (I) ou (II) : 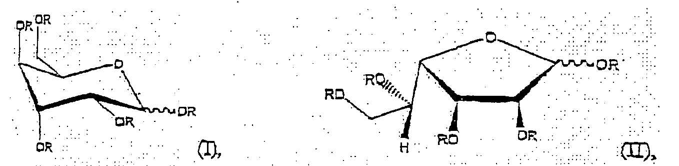 dans lesquelles chaque R est indépendamment H, un groupe alpha-cétoalcanoate ou un acyle en C₁-C₃ et au moins un R est un groupe alpha-cétoalcanoate.

20. Utilisation selon la revendication 6, dans laquelle ledit ester d'acide alpha-cétoalcanoïque est un ester de dihydroxyacétone.

21. Utilisation selon la revendication 6, dans laquelle ledit ester d'acide alpha-cétoalcanoïque est un thiolester.

22. Utilisation selon la revendication 21, dans laquelle une partie thiol dudit thiolester est de la cystéine ou de l'homocystéine.

23. Utilisation selon la revendication 1, dans laquelle un amide d'un acide alpha-cétoalcanoïque est utilisé.

24. Utilisation selon la revendication 23, dans laquelle ledit amide d'un acide alpha-cétoalcanoïque est un pyruvamide.

25. Utilisation selon la revendication 23, dans laquelle ledit amide est un amide d'acide aminé d'un acide alpha-cétoalcanoïque.

26. Utilisation de pyruvate d'éthyle dans la fabrication d'un médicament pour traiter une hépatite alcoolique.

27. Utilisation selon la revendication 26, dans laquelle l'hépatite alcoolique est une hépatite alcoolique aiguë.

28. Utilisation selon la revendication 26, dans laquelle l'hépatite alcoolique est une hépatite alcoolique chronique.

29. Ester d'un acide alpha-cétoalcanoïque ou un amide d'un acide alpha-cétoalcanoïque pour une utilisation dans le traitement d'une hépatite chez un sujet.

30. Ester selon la revendication 29, dans lequel un ester d'un acide alpha-cétoalcanoïque est utilisé et ledit ester est le pyruvate d'éthyle.

31. Ester selon la revendication 29, dans lequel un ester d'un acide alpha-cétoalcanoïque en C₃-C₈ à chaîne linéaire ou branchée est utilisé et ledit ester est contenu dans une solution saline isotonique de Ringer.

32. Pyruvate d'éthyle pour une utilisation dans le traitement d'une hépatite alcoolique.
